# EUROPEAN PATENT SPECIFICATION

(11) **EP 2 682 076 B1**
(45) Date of publication and mention of the grant of the patent: **23.12.2015**
(21) Application number: 13175310.5
(22) Date of filing: 05.07.2013
(51) Int. Cl.: A61F 9/007, C12N 5/071

(54) **Surgical instrument**
Chirurgisches Instrument
Instrument chirurgical

(30) Priority: 05.07.2012 JP 2012151924
(43) Date of publication of application: 08.01.2014
(73) Proprietor: Nidek Co., Ltd., Aichi 433-0038 (JP)
(72) Inventor: Mita, Osamu, Aichi, 443-0038 (JP)
(74) Representative: Stöckeler, Ferdinand

(56) References cited:
- US-A1- 2003 216 747
- US-A1- 2004 039 401
- US-A1- 2007 208 422
- US-A1- 2008 281 341
- US-A1- 2010 211 079

## Description

### BACKGROUND

### 1. Technical Field

The present disclosure relates to a surgical instrument.

### 2. Related Art

In recent years, a treatment method has been proposed with respect to regenerative medicine in the ophthalmic field. The method includes culturing retinal pigment epithelial cells to give the cells in a sheet-like form and transplanting the epithelial cells beneath the retina of the fundus of a patient's eye. Specifically, an example of such a method is described in JP-A-9-501303. However, there is difficulty in surgery to transplant (implant) a produced sheet-like graft beneath the retina of the fundus of the patient's eye. For facilitating the transplant surgery, a surgical instrument has been proposed in, for example, U.S. Patent No. 6159218. According to the patent document, a graft is pushed out of the tip of a nozzle (a tubular member) of a surgical instrument to beneath a treatment area, the retina of the fundus, and transplanted beneath the retina. In such a case, a through hole (incision) through which the distal end part of the surgical instrument is inserted is provided to the eyeball of a patient's eye.

US 2008/281341 A1 discloses an apparatus for endothelial keratoplasty donor tissue transport and delivery to a recipient's eye. The apparatus comprises an elongated apparatus body upon which a sleeve is slidably received. A tissue holder is attached to the apparatus body and a tip provided at a distal end of the sleeve is movable relative to the tissue holder through respective movement of the sleeve and the apparatus body.

### SUMMARY

A surgical instrument includes: a handpiece; a rod part at a distal end of the handpiece, the rod part including a surface for adhering a graft thereto; a hollow pipe part configured to cover the rod part, the pipe part including an inner diameter with a predetermined clearance between the pipe part and the rod part; and a moving unit configured to move the pipe part relative to the rod part along an axial direction of the rod part.

### BRIEF DESCRIPTION OF DRAWINGS

FIG. 1 is an exterior perspective view of an ophthalmic surgical instrument according to an embodiment of the present disclosure;
FIG. 2A is a side view of the ophthalmic surgical instrument;
FIG. 2B is a cross-sectional side view of the ophthalmic surgical instrument;
FIG. 2C is a cross-sectional side view of the surgical instrument illustrated in FIG. 2B, where a plunger has been moved;
FIG. 3 is a cross-sectional view of a vicinity of a rotating knob of the ophthalmic surgical instrument;
FIG. 4A is an upper surface cross-sectional view of a state of a nozzle unit before a pipe part is moved with respect to a rod;
FIG. 4B is an upper surface cross-sectional view of a state of the nozzle unit after the pipe part is moved with respect to the rod;
FIG. 5 is a front view of a distal end part of the nozzle unit;
FIG. 6A is a diagram illustrating a graft and the distal end part of the nozzle unit, which has been brought close to the graft;
FIG. 6B is a diagram illustrating the graft and the distal end part of the nozzle unit, which is mounting the graft thereon; FIG. 7A is a diagram illustrating space formed beneath the retina; FIG. 7B is a diagram illustrating the nozzle unit inserted beneath the retina; and FIG. 7C is a diagram illustrating the graft discharged beneath the retina.

### DETAILED DESCRIPTION

In the following detailed description, for purpose of explanation, numerous specific details are set forth in order to provide a thorough understanding of the disclosed embodiments. It will be apparent, however, that one or more embodiments may be practiced without these specific details. In other instances, well-known structures and devices are schematically shown in order to simplify the drawing.

In the surgical instrument described in U.S. Patent No. 6159218, a predetermined member presses a graft from the back. Consequently, the graft is pushed out of the instrument. However, such an instrument makes the graft and the instrument come into mechanical contact with each other when the graft is pushed out. Hence, the graft may be damaged. Moreover, there is a difficulty in suitably performing the operation of pushing out the graft.

Moreover, a through hole to be formed in a patient's eye is desirable to be as small as possible. However, if a large graft is transplanted in the patient's eye, the following trouble may occur with the surgical instrument described in U.S. Patent No. 6159218. In other words, with this surgical instrument, the size of a nozzle for discharging a graft increases depending on the size of the graft. If the size of the nozzle increases, a through hole to be formed in the patient's eye is enlarged. If the through hole is enlarged, it places a burden on the patient.

An object of the present disclosure is to provide a surgical instrument according to claim 1 that can suitably transplant a large graft in the eye of a patient while avoiding any damage to the graft without any burden on a patient.

A surgical instrument includes: a handpiece; a rod part at a distal end of the handpiece, the rod part including a surface for adhering a graft thereto; a hollow pipe part configured to cover the rod part, the pipe part including an inner diameter with a predetermined clearance between the pipe part and the rod part; and a moving unit configured to move the pipe part relative to the rod part along an axial direction of the rod part.

This surgical instrument can suitably transplant a large graft in the eye of a patient while avoiding any damage to the graft without any burden on a patient.

Hereinafter, an embodiment of the present disclosure will be described with reference to the accompanying drawings. In the following, an ophthalmic surgical instrument used in transplant surgery to transplant (implant) a graft beneath the retina of a patient's eye will be described as an example of the surgical instrument according to the embodiment. This ophthalmic surgical instrument includes a member for adhering a graft thereto and placing the graft beneath the retina using fluid.

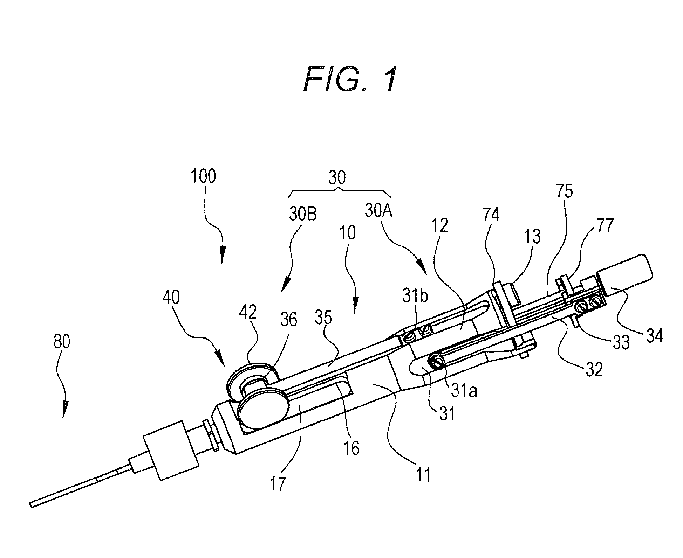

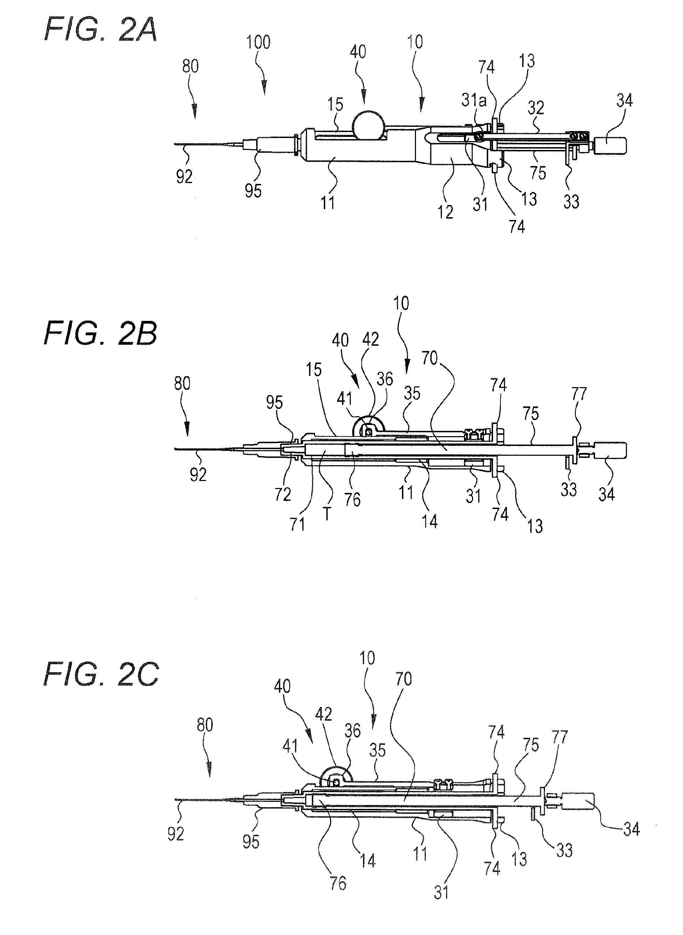

FIG. 1 is an exterior perspective view of an ophthalmic surgical instrument (hereinafter simply abbreviated to a surgical instrument) 100 according to the embodiment. FIG. 2A is a side view of the surgical instrument 100. FIGS. 2B and 2C are cross-sectional side views of the surgical instrument 100.

The surgical instrument 100 mainly includes a repeatedly usable (reusable) handpiece 10 and a disposable syringe 70 and nozzle unit 80. The materials of the handpiece 10 can be autoclaved. The handpiece 10 includes, for example, durable materials that can be used several times. The materials of parts of the handpiece 10 are for example, a corrosion-resistant metal (e.g., stainless steel or titanium). For example, a disposable (one use) type injection syringe in circulation for a medical application is suitably used as the syringe 70. The nozzle (nozzle unit) 80 is detachable from the syringe 70. FIG. 2A is a side view of the surgical instrument 100. FIG. 2B is a cross-sectional side view of obtained by cutting the surgical instrument 100 illustrated in FIG. 2A at its central axis. FIG. 2C is a cross-sectional side view of the surgical instrument 100 illustrated in FIG. 2B, where a plunger 75 has moved forward.

The syringe 70 of the embodiment is a medical injection syringe. The syringe 70 includes an outer tube 71 and the plunger (extrusion rod) 75. The outer tube 71 includes a distal end part 72 for attaching the nozzle unit 80 thereto. An opening for fitting the plunger 75 thereinto is formed at a rear end of the outer tube 71. The outer tube 71 is detachable from the handpiece 10. A pair of flanges (brims) 74 is formed on the outer periphery of the rear end of the outer tube 71.

An elastic member 76 is provided at a distal end of the plunger 75. The elastic member 76 is formed of, for example, rubber or resin. A press part 77 to be pressed by the finger of an operator, or the like is provided at a rear end of the plunger 75. The plunger 75 is fitted into the outer tube 71 so as to seal the opening part of the outer tube 71 with the elastic member 76. Consequently, an internal space of the outer tube 71 (the syringe 70) serves as a tank (tank part T) for accommodating fluid. The plunger 75 can be longitudinally (axially) moved along the outer tube 71. In the embodiment, the distal end part 72's side of the syringe 70 is defined as a front side thereof in the longitudinal direction. Therefore, the plunger 75 moves in the front-back direction.

If the plunger 75 moves forward in the outer tube 71, the capacity of the tank part T reduces. On the other hand, if the plunger 75 moves backward in the outer tube 71, the capacity of the tank part T increases. A positive pressure or negative pressure is generated in the syringe 70 (the tank part T) with a change in the capacity of the tank part T. The plunger 75 serves as a suction/drain unit for sucking fluid into the tank part T or draining the fluid from the tank part T. Fluid is sucked from the nozzle unit 80 and the distal end part 72 into the tank part T or drained from the tank part T with a front-back movement of the plunger 75 (a change in the capacity of the tank part T). In the embodiment, the fluids to be accommodated in the tank part T include, for example, ophthalmic perfusate (normal saline). The fluid may be, for example, a viscoelastic material or a medicine for ophthalmic use.

The handpiece 10 includes a grip part 11, a guide pipe 12, a fixed part 13, an inner tube part 14, a moving unit 30, and a rotating knob 40. The grip part 11 is an outer tube of the handpiece 10, and is a portion to be held by the fingers of an operator (a handle part). The guide pipe 12 guides the movement of the moving unit 30. The fixed part 13 fixes (holds) the syringe 70 to the handpiece 10. The inner tube part 14 is an inner tube for housing the syringe 70 in the handpiece 10. The moving unit 30 is a member for moving the plunger 75. The rotating knob 40 is an operating member (operating unit) for the operator to operate the moving unit 30. The rotating knob 40 is a part of the moving unit 30.

A ring 31 of the moving unit 30, which is described below, is slidably attached to an inside of the guide pipe 12. Moreover, two different slits are axially formed in the guide pipe 12 along the axial direction. These slits allows the ring 31to be connected to both a bar 32 and a slide bar 35, which are described below.

The fixed part 13 is provided at a rear end of the handpiece 10. The fixed part 13 includes a guide hole that firmly holds the flanges 74. If the syringe 70 rotates, the flanges 74 are firmly held by the guide hole. Consequently, the flanges 74 are fixed by the guide hole to the fixed part 13 to prevent their front-back movements. The fixed part 13 is a detachable member for detachably fixing to the handpiece 10 the syringe 70 including the tank part T.

The outer tube 71 is housed in the inner tube part 14. An opening for inserting (exposing) the distal end part 72 of the syringe 70 therethrough is provided at a distal end of the inner tube part 14. The size of the opening is defined such that the syringe 70 does not come out. An opening is formed at a rear end of the fixed part 13. The outer tube 71 is loaded and unloaded from this opening.

The moving unit 30 mainly includes a holder 30A, the rotating knob 40, and a slider 30B. The holder 30A holds the press part 77 of the plunger 75. The rotating knob 40 is a rotating member for the operator to operate. The slider 30B moves the holder 30A with the rotation of the rotating knob 40. The holder 30A and the slider 30B share the ring 31. The ring 31 is movably arranged in the guide pipe 12. In the moving unit 30, the ring 31 is a proximal part. The holder 30A is provided backward of the ring 31. The slider 30B is provided forward of the ring 31. The holder 30A includes the bar 32, a plate 33, and a rotating pin 34. The bar 32 is rotatably held around the axis by a pin 31a attached to the ring 31. The plate 33 is provided at a rear end of the bar 32. The rotating pin 34 fixes and holds the press part 77.

The bar 32 rotates about the pin 31a portion as a rotation axis in an up-down direction of the handpiece 10 from a state of being positioned in a parallel direction with the plunger 75 (the axial direction), the state being illustrated in FIG. 2A. The plate 33 includes a female screw. The rotating pin 34 includes a male screw. The rotating pin 34 is threadedly engaged by these screws with the plate 33. The entire rotating pin 34 moves in the front-back direction by the axial rotation of the rotating pin 34.

The press part 77 is arranged between the plate 33 and the rotating pin 34. If the rotating pin 34 is rotated in one direction to be advanced forward, the press part 77 is sandwiched between the plate 33 and the rotating pin 34. Consequently, the plunger 75 is fixed to and held by the holder 30A.

If the plunger 75 is removed from the holder 30A, the rotating pin 34 is rotated in the reverse direction and moved backward. The plate 33 and the bar 32 are subsequently rotated downward. Moreover, after the fixation of the flange 74 is released, the plunger 75 is pulled out of the inner tube part 14.

The slider 30B includes the ring 31, the slide bar 35, and a hook 36. The slide bar 35 has a linear shape. The slide bar 35 is screwed by a screw 31b to the ring 31. The hook 36 is provided at a distal end of the slide bar 35 and rotatably holds the rotating knob 40. Moreover, a guide rail 15 and a wall part 16 are formed in an upper portion of the grip part 11. The guide rail 15 guides the movement of the rotating knob 40 (the slide bar 35). The wall part 16 includes opposed side walls for supporting both side surfaces of the rotating knob 40. The guide rail 15 and the wall part 16 are linearly formed in the front-back direction (axial direction) of the grip part 11.

The hook 36 is provided at a distal end of the slide bar 35. The hook 36 is hooked by a shaft 41 of the rotating knob 40, the shaft 41 being described below. In other words, the hook 36 can rotate the rotating knob 40. Furthermore, the hook 36 has a function of moving the slide bar 35 in the axial direction by rotating the rotating knob 40.

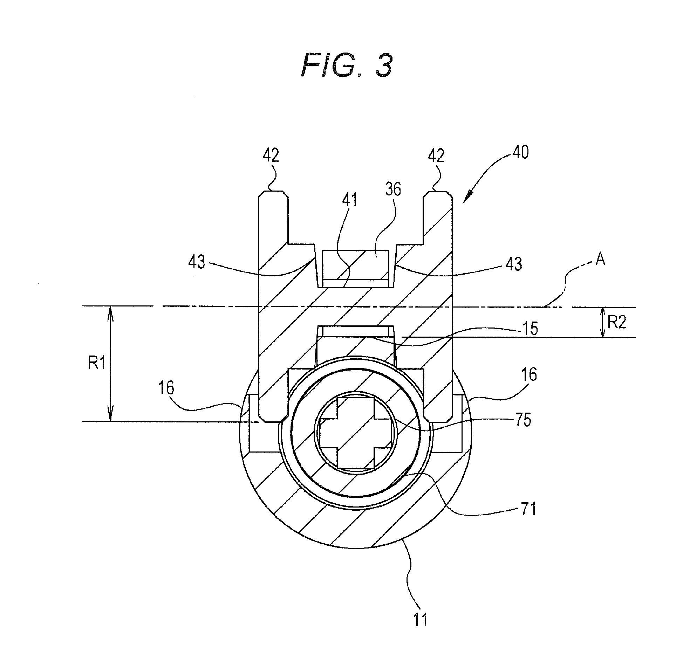

FIG. 3 is a diagram illustrating the configuration of the rotating knob 40. The rotating knob 40 includes the shaft 41, a disk part 42, and an inclined part 43. The disk part 42 is provided on both sides of the shaft 41, and has a larger outer diameter than the shaft 41. The inclined part 43 is inclined from the shaft 41 toward an outer circumferential direction of the disk part 42. The rotating knob 40 is an integral member. The inclined part 43 is a portion of the disk part 42, where its thickness progressively reduces from the shaft 41 toward the outer circumferential direction of the disk part 42. In other words, the inclined part 43 has a tapered shape. The width of the shaft 41 (the length of the shaft 41 in the axial direction) is designed such that the shaft 41 does not come into contact with an upper surface of the guide rail 15 when the inclined part 43 comes into contact with a corner of the guide rail 15. Therefore, in the rotating knob 40, the inclined part 43 comes into contact with the guide rail 15.

The rotating knob 40 can move back and forth along the guide rail 15 and the wall part 16 with the rotation of the disk part 42. The slider 30B (the hook 36, the slide bar 35, and the ring 31) and the holder 30A (the ring 31, the bar 32, the plate 33, and the rotating pin 34) move integrally with the movement of the rotating knob 40 in the front-back direction. Consequently, the plunger 75 moves in the front-back direction. At this point, the outer tube 71 is fixed and held by the fixed part 13. Therefore, the plunger 75 moves in the front-back direction relative to the outer tube 71.

The amount of movement of the rotating knob 40 is proportional to the amount of rotation of the rotating knob 40 (the disk part 42). In the embodiment, a predetermined portion (the inclined part 43) of the rotating knob 40 comes into contact with the guide rail 15. The outer diameter of the inclined part 43 is smaller than the diameter of the disk part 42 that is the largest outer diameter of the rotating knob 40. Therefore, the actual amount of movement of the rotating knob 40 in the front-back direction is smaller than the amount of rotation of the disk part 42. As illustrated in FIG. 3, the distance between a rotation axis A of the shaft 41 and a peripheral edge of the disk part 42 is defined as R1 (corresponding to the radius of the largest outer diameter of the rotating knob 40). The distance between the rotation axis A and the guide rail 15 is defined as R2. The ratio, at this point, of the amount of movement of the rotating knob 40 in the front-back direction by the rotation of the disk part 42 to the amount of movement of the disk part 42 when the disk part 42 rotates on a predetermined plane is the ratio of the distance R2 to the distance R1 (R2/R1).

In this manner, the amount of rotation of the rotating knob 40 (the disk part 42) is converted into the reduced amount of linear movement (the amount of rolls of the rotating knob 40), and transferred to the slider 30B, the holder 30A, and the plunger 75. Such a mechanism facilitates fine adjustments for operation on the fluid in the tank part T. Therefore, the operability of sucking and draining the fluid via the nozzle unit 80 is improved.

As illustrated in FIGS. 2B and 2C, the rotating knob 40 moves forward by rotation without changing its height position with respect to the grip part 11. Accompanied by the movement of the shaft 41 of the rotating knob 40, the member integrated with the hook 36, the slide bar 35, and the like (the slider 30B) moves forward. Consequently, the plunger 75 moves forward and reduces the capacity of the tank part T, while the fluid in the tank part T is drained to the outside. If the direction of rotation of the rotating knob 40 is a direction moving backward, the fluid is sucked (flowed) into the tank part T, opposite to the above-mentioned operation.

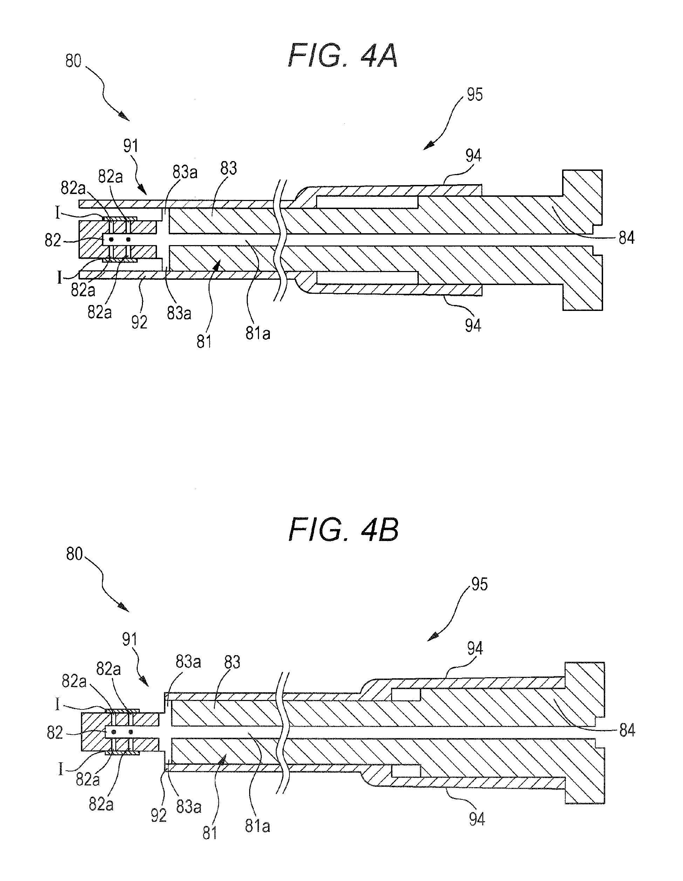

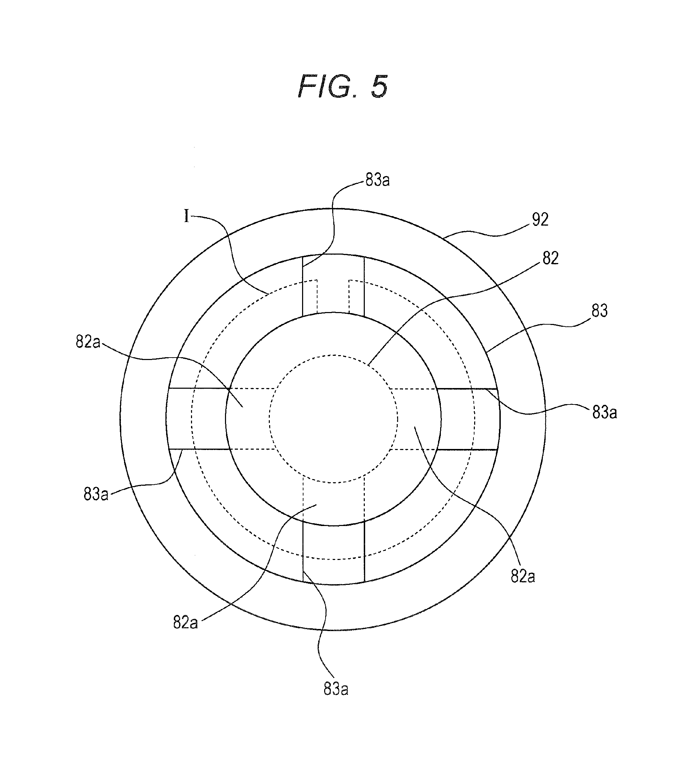

Next, the nozzle unit 80 to be attached to the syringe 70 will be described with reference to the accompanying drawings. FIGS. 4A and 4B are upper surface cross-sectional views of the nozzle unit 80 (cross-sectional views on a horizontal plane along the axis of the nozzle unit 80). FIG. 5 is a front view of a distal end part of the nozzle unit 80. In FIG. 5, a graft I is illustrated by a dotted line.

The nozzle unit 80 includes a rod part 81 and a pipe part 91. The graft I is adhered to (wound around) the rod part 81, and subsequently separated from (peeled off) the rod part 81. In this manner, the rod part 81 is a member for transplanting the graft I (for example, implanting the graft I in the fundus of the patient's eye). The rod part 81 is a rod-shaped member. The pipe part 91 protects the graft I by covering the graft I adhered to the rod part 81. The pipe part 91 has a function of covering the graft I. The inner diameter of the pipe part 91 is defined so as to provide predetermined clearance between the pipe part 91 and the rod part 81. A rotating part 95 is arranged at a proximal end (rear end) of the nozzle unit 80. The rotating part 95 includes a proximal end part (a proximal part 94) of the pipe part 91 and a proximal end part (a proximal part 84) of the rod part 81, the proximal end part being threadedly engaged with the pipe part 91 (the proximal part 94). The rotating part 95 is a moving unit for moving the pipe part 91 along the axial direction of the rod part 81. FIG. 4A illustrates a state of the nozzle unit 80, where the pipe part 91 has moved to the front (where the pipe part 91 is covering the entire rod part 81). FIG. 4B illustrates a state of the nozzle unit 80, where the pipe part 91 has moved (withdrawn) to the rear (where the rod part 81 is exposed from the pipe part 91).

The rod part 81 includes a distal end part 82, a support part 83, and the proximal part 84. The distal end part 82 is a member for adhering a graft thereto. The support part 83 is connected to a rear end of the distal end part 82, and supports the distal end part 82. The rear end of the support part 83 is fixed to the proximal part 84. The cross-sectional shapes (shapes of cross sections orthogonal to the axial direction) of the distal end part 82 and the support part 83 are both a hollow circle. A distal end portion of the distal end part 82 is closed.

The outer diameter of the distal end part 82 is designed such that the graft I adheres to a side surface of the distal end part 82. It is preferred that the outer diameter dimension of the distal end part 82 be as small as possible. In the embodiment, the outer diameter dimension of the distal end part 82 is designed such that both ends of the graft I do not overlap each other and both ends come into proximity to each other when the graft I is adhered to and wound around the side surface of the distal end part 82. In other words, the outer diameter of the distal end part 82 is defined so that the graft I will have a C-shape when viewed from the axial direction of the distal end part 82. In other words, the circumference of the distal end part 82 (the distal end of the rod part 81) is longer than one side of the graft.

The support part 83 has stiffness to the degree that the entire rod part 81 does not bend excessively. It is preferred that the outer diameter dimension of the support part 83 be formed to be as small as possible. The support part 83 can prevent the graft I adhered to the distal end part 82 from coming into contact with the pipe part 91 even if the pipe part 91 (which is described below) has axial run-out. Therefore, It is preferred that the length of the distal end part 82 in the axial direction be short.

The difference (step height) between the outer diameter of the distal end part 82 and the outer diameter of the support part 83 is defined in a predetermined range. The predetermined range is defined so that the graft I adhered to the distal end part 82 can be prevented from coming into contact with an inner wall of the pipe part 91, and that the outer diameter of the support part 83 can be made as small as possible. In other words, the difference in diameter between the distal end part 82 and the support part 83 is defined to ensure space that can accommodate a graft (a step height greater than the thickness of the graft) in a state where the graft is adhering to the distal end part 82. The proximal part 84 is located at the proximal end of the support part 83. A fitting portion (opening) to fix the nozzle unit 80 to the syringe 70 is provided at a rear end of the proximal part 84. The proximal part 84 has a role in supporting the nozzle unit 80. A screw thread for threadedly engaging the proximal part 94 of the pipe part 91 is formed in the outer peripheral surface of the proximal part 84.

The pipe part 91 includes a tube part (pipe part) 92 and the proximal part 94. The tube part 92 is a hollow tube-shaped (pipe-shaped) member. The proximal part 94 is connected to a proximal end of the tube part 92, and is responsible for supporting the tube part 92. A screw thread that threadedly engages the proximal part 84 of the rod part 81 is formed on an inside of the proximal part 94. The pipe part 91 is a hollow member having a larger inner diameter than the outer diameter of the rod part 81. The cross-sectional shape of the tube part 92 is a circle similarly to the support part 83 of the rod part 81. The inner diameter of the tube part 92 is equivalent to or slightly larger than the outer diameter of the support part 83 of the rod part 81. Consequently, it becomes easy for the pipe part 91 to move with respect to the rod part 81. The tube part 92 is inserted into the eyeball of the patient's eye. Consequently, It is preferred that the outer diameter of the tube part 92 be as small as possible. The cross-sectional shapes of the rod part 81 (the distal end part 82 and the support part 83) and the pipe part 91 (the tube part 92) are not necessarily a circle, but may be a shape that allows a graft to adhere thereto or the pipe part 91 to move. For example, these cross-sectional shapes may be a polygon, a star shape, or the like. Moreover, these cross-sectional shapes may be an ellipse.

The rotating part 95 includes the proximal part 84, and the proximal part 94 that threadedly engages the proximal part 84. The rotation of the proximal part 94 rotates the entire pipe part 91. Consequently, the pipe part 91 performs advance and retreat movements (sliding movements) along the axial direction of the rod part 81. The tube part 92 of the pipe part 91 moves a predetermined distance such that at least two states where the distal end part 82 is exposed from the tube part 92 (the state illustrated in FIG. 4B) and where the distal end part 82 is covered by the tube part 92 (the state illustrated in FIG. 4A) are realized. In other words, the pipe part 91 moves from a position where a distal end of the pipe part 91 is arranged in the vicinity of the border between the distal end part 82 and the support part 83 of the rod part 81 to a position where the distal end of the pipe part 91 is in line with a most distal end of the distal end part 82.

In the embodiment, when the graft I is adhered to the distal end part 82, the rotating part 95 is rotated to retreat (withdraw) the pipe part 91 and expose the distal end part 82 (see FIG. 4B). After the graft I is adhered to the distal end part 82, the rotating part 95 is reversely rotated to move the pipe part 91 forward (see FIG. 4A). In this manner, the rotation operation (forward/reverse rotation) is performed on the rotating part 95. Accordingly, the pipe part 91 performs advance and retreat movements. Consequently, the state of the pipe part 91 is switched between the two states of the state illustrated in FIG. 4A and the state illustrated in FIG. 4B. Consequently, the graft I is protected by the tube part 92 (see FIG. 4A). The transplant work of the graft I is performed in the state where the graft I is being protected.

Assume here that the shape of the graft I is, for example, a 3 mm × 3 mm square sheet shape having a thickness of several tens µm. The diameter of the distal end part 82 is assumed to be, for example, approximately 1 mm (slightly more than 3 mm as a circumference). The length of the distal end part 82 in the axial direction is assumed to be at least more than 3 mm (e.g., 4 mm to 10 mm). The outer diameter of the support part 83 is assumed to be, for example, 1.4 mm (the step height between the distal end part 82 and the support part 83 is 200 µm). The inner diameter of the tube part 92 is assumed to be, for example, 1.4 mm. The outer diameter of the tube part 92 is assumed to be, for example, 1.7 mm. In this case, the outer diameter of the nozzle unit 80 to be inserted into the eye is 1.7 mm.

As illustrated in FIGS. 4A, 4B, and 5, the rod part 81 of the embodiment is an integrally formed hollow rod-shaped member. A liquid flow passage 81a is formed inside the rod part 81. The flow passage 81a is formed from the proximal part 84 of the rod part 83 toward the distal end part 82. A drain hole 82a linked to the flow passage 81a is formed on the side surface of the distal end part 82 of the rod part 81. Moreover, a drain hole 83a linked to the flow passage 81a is formed on a front surface of the support part 83 (an end surface on the distal end part 82 side). Fluid is drained from the rod part 81 through the drain holes.

FIG. 5 illustrates a front view of the distal end part of the nozzle unit 80. An upper side in a vertical direction (an up-down direction when in use) of the distal end part 82 when viewing the distal end part 82 from the front is defined to be a reference. In this case, two drain holes 82a are formed on a lower side in the vertical direction, and two in each of left and right directions orthogonal to the vertical direction. The drain holes 82a are arranged in twos along the axial direction (see FIGS. 4A and 4B). It is sufficient if the drain hole 82a is located at a position that the graft I comes off the distal end part 82 when the liquid to be drained from the drain hole 82a presses the graft I adhered to the distal end part 82. It is preferred that the drain hole 82a be formed beneath the graft I (at a position to be covered by the graft I). In the embodiment, the drain hole 82a is formed at a position that does not inhibit the graft I from coming off the distal end part 82. In other words, the drain hole 82a is not provided in an area where both ends (and the boundary between both ends) of the graft I adhered to the distal end part 82 are located (the upper side in the vertical direction of the distal end part 82).

The shape of the drain hole 82a is formed so as to suppress that the graft I or part thereof enters inside the distal end part 82, or that the graft I is damaged by an edge of the drain hole 82a. In the embodiment, the shape of the drain hole 82a is a circle with a diameter of several tens to several hundreds µm. It is preferred that an edge of the drain hole 82a be chamfered (or rounded).

Liquid flow (a flow passage) for separating the graft I adhered to the distal end part 82 from the distal end part 82 and pushing out the graft I forward is created by the drain hole 83a. One drain hole 83a is arranged in each of up, down, left and right directions with respect to the vertical direction. In other words, the drain hole 83a is arranged at four points. The hole shape of the drain hole 83a is substantially similar to that of the drain hole 82a. The drain holes 83a are formed along a direction orthogonal to the axis.

A drain hole linked to the flow passage 81a is not formed at the most distal end portion (the distal end in the axial direction) of the distal end part 82. Hence, part of fluid flowing through the flow passage 81 a flows from the side surface of the distal end part 82 to the outside through the drain holes 82a. Moreover, the other part of the fluid flowing through the flow passage 81a flows from the front surface of the support part 83 to the outside through the drain holes 83a.

The shapes of the drain holes 82a and 83a may be any hole shape as long as their adverse effects on the graft I are small. The shapes of these drain holes 82a and 83a may be, for example, a square hole or a slit hole. Moreover, it is sufficient if the positions and numbers of the drain holes 82a and 83a are the positions and numbers that enable the graft I to come off the distal end part 82 and be discharged from the nozzle unit 80. The drain holes 82a and 83a may be formed at least either of the distal end part 82 and the support part 83. Moreover, there may be formed at least one drain hole 82a and one drain hole 83a. Moreover, either of the drain holes 82a and 83a may be formed.

The material of the rod part 81 and the pipe part 91 may be, for example, a material having biocompatibility. Moreover, it is preferred that the material of the distal end area of the tube part 92 (a region where the distal end part 82 is accommodated) could be, for example, a material being light transmissive. Consequently, even if the distal end part 82 is covered by the tube part 92, it becomes easier to visually confirm a state of the graft I adhered to the distal end part 82. The material of the rod part 81 may be, for example, a metal such as stainless, titanium, or platinum, or a resin such as polypropylene. The material of the pipe part 91 may be, for example, a resin such as fluorine resin, polypropylene, or silicone.

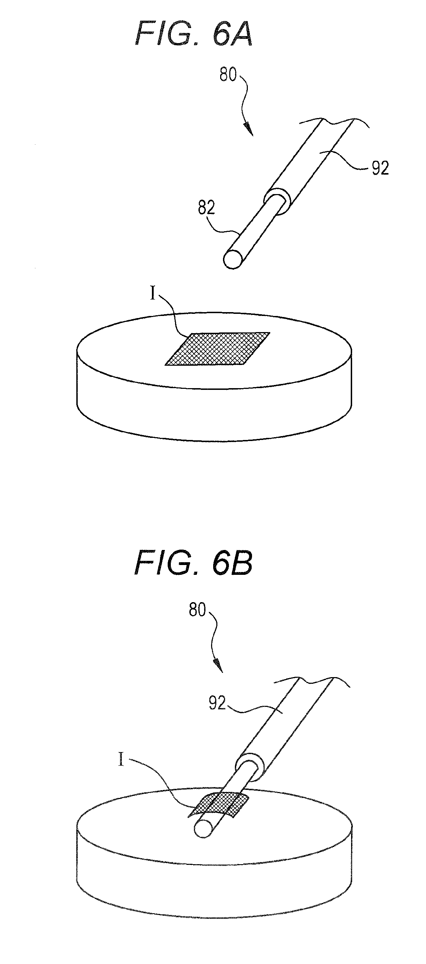

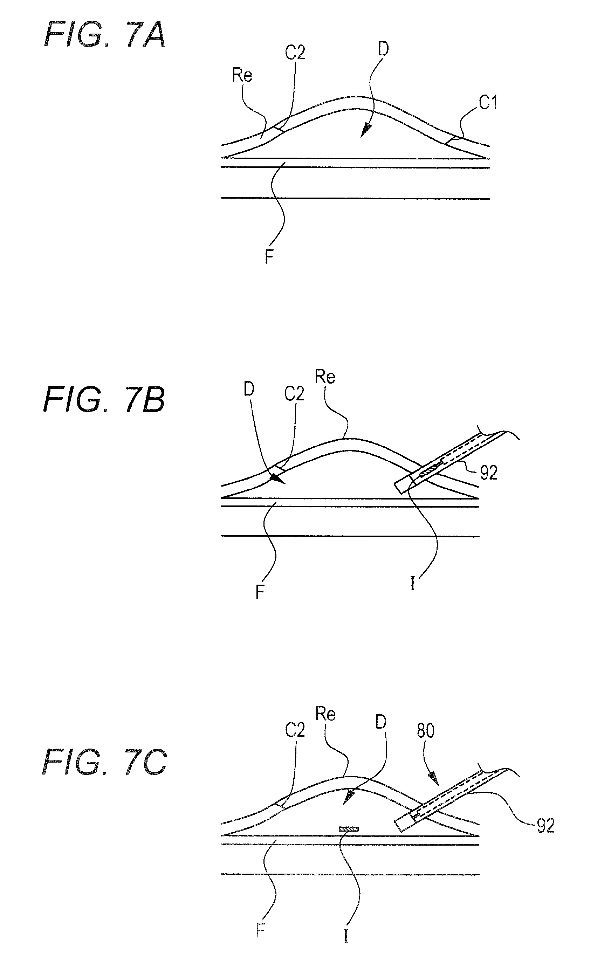

A description will be given of the operation of the surgical instrument 100 having the above configuration. FIGS. 6A and 6B are diagrams illustrating the operation of adhering the graft I to the distal end part 82. FIGS. 7A, 7B, and 7C are diagrams illustrating the transplant work of the graft I. The graft I of the embodiment is a sheet-shaped cell sheet. The graft I is obtained by differentiating tissue previously removed from a patient into retinal pigment epithelial cells and culturing the differentiated retinal pigment epithelial cells. The graft I is cut into a small piece (e.g., 3 × 3 mm) and transplanted (implanted) beneath the retina of the fundus of the patient's eye using the surgical instrument 100.

An operator (or an assistant) assembles the syringe 70, the handpiece 10, and the nozzle unit 80. The syringe 70 is inserted into the handpiece 10 and fixed there. The rotating knob 40 and the plunger 75 are assembled to work in an interlocked fashion. The nozzle unit 80 is fixed to the syringe 70, while the distal end part 82 is exposed (see FIG. 4B). The operator operates the rotating knob 40 to store perfusate in the tank part T, while the perfusate is taken in the tank part T through the distal end part 82. Consequently, the surgical instrument 100 becomes ready to be used.

The operator adheres the graft I previously processed into a small piece to the distal end part 82. As illustrated in FIG. 6A, the graft I that has become a small piece is floating on a culture medium in a Petri dish. The graft I is cut from the culture sheet and placed at a fluid surface with a basement membrane side up. The operator changes the orientation of the surgical instrument 100, for example, by turning the surgical instrument 100 upside down. The operator places the distal end part 82 of the surgical instrument 100 beneath the graft I. As illustrated in FIG. 6B, the graft I is then scooped up by the distal end part 82. At this point, It is preferred that the operator adjust the position of the distal end part 82 such that the top of the side surface of the distal end part 82 (the central portion of the distal end part 82) coincides with the center of the graft I. If the operator further moves the distal end part 82 upward from the state of FIG. 6B, the graft I acts in a manner of winding around the side surface of the distal end part 82 due to the surface tension of the culture medium. In this manner, as illustrated in FIG. 5 (the top and bottom are reversed in FIG. 5), the graft I is adhered to the distal end part 82, while the basement membrane side of the graft I is an outer side (outer circumference side).

The operator may adhere the graft I to the distal end part 82 by rotating the surgical instrument 100 around the axis. Moreover, the operator may cause the graft I to stick to the side surface of the distal end part 82 by operating the rotating knob 40. Moreover, the operator may bring the graft I into intimate contact with the distal end part 82 by absorbing water from the graft I adhered to the distal end part 82 (or the surroundings of the graft I) with absorbent paper and the like.

If the rotating part 95 (the proximal part 94) is rotated, the tube part 92 is moved forward. Consequently, the graft I is covered by the tube part 92. Consequently, even if the graft I is inserted into the eye, the graft I is hardly damaged.

In transplant surgery, a through hole is formed in the sclera of the anterior segment of the patient's eye. Forceps and the like are inserted into the eye from this through hole. As illustrated in FIG. 7A, a domical space D is formed beneath the retina of the fundus of the patient's eye, in other words, between a retina (sensory retina) Re and fundus tissue F other than the retina. The space D is formed by injecting perfusate or the like from an incision C1 formed by cutting part of the retina Re. An incision C2 different from the incision C1 is also formed. The incisions C1 and C2 are formed to face each other. Part of the perfusate or the like that has been injected from the incision C1 into the space D flows out from the incision C2. Consequently, a burden (load) created on the retina Re and the like by addition of the pressure and the like of the perfusate can be reduced. Next, the operator removes an unnecessary newborn blood vessel, pigment epithelial cell, and the like in the space D.

As illustrated in FIG. 7B, the operator inserts the distal end of the nozzle unit 80 into the space D. As illustrated in FIG. 7C, the operator discharges the graft I. The operator sends the perfusate to the distal end of the nozzle unit 80 by rotating the rotating knob 40 forward. The graft I is pushed by the perfusate to be drained from the drain hole 82a and comes off the distal end part 82. Moreover, the graft I is pushed by the perfusate to be drained from the drain hole 83a and moves forward. Consequently, the graft I adhered in curl to the distal end part 82 is spread and discharged from the distal end of the nozzle unit 80 to the outside.

The operator places the graft I at a transplant position, and removes the nozzle unit 80 from inside the eye. The operator sucks and removes the perfusate in the space D and returns the retina Re to its original state. In this manner, the graft I is transplanted in the patient's eye. The operator may retreat the pipe part 91 in the space D when discharging the graft I.

As described above, according to the surgical instrument 100, the large graft I can be suitably transplanted in the patient's eye while any damage to the graft I can be avoided without any burden on a patient. In the surgical instrument 100, the graft I is moved using fluid. Consequently, damage to the graft I can be suppressed. Upon adhesion of the graft I, the graft I is wound around the side surface of the distal end part 82 (the graft I is curled). Consequently, the size of a unit housing the graft I can be reduced. Consequently, an incision to be formed in the patient's eye can be made small. Moreover, the movement of the graft I during transplant work is suppressed by adhering the graft I to the distal end part 82. Consequently, transplant work becomes easy.

In the above description, the pipe part 91 moves with respect to the rod part 81. However, the moving unit (the pipe part 91 and the rod part 81) is not limited to this, but it is sufficient if the moving unit is configured such that the pipe part 91 moves relative to the rod part 81 and can protect the graft I adhered to the distal end part 82. For example, the rod part 81 may be configured to move by a rotation operation. Moreover, it is sufficient if the moving unit is configured such that the pipe part 91 or the rod part 81 can be moved regardless of a rotation operation. For example, the moving unit may include a slider mechanism. Moreover, the pipe part 91 as a cap may cover the rod part 81 from the front to cover the distal end portion of the rod part 81. In this case, the cap serves as both the pipe part and the moving unit. In this case, the cap is removable. It is also possible to remove the cap from the rod part 81 after covering the graft I with the cap.

In the above description, the nozzle unit 80 is a linear member. However, the nozzle unit 80 is not limited to this, but it is sufficient if the nozzle unit 80 is configured to be capable of adhering the graft I to the distal end part 82 and covering the graft I with the pipe part 91. For example, the nozzle unit 80 may include the rod part 81 that is bent in the middle. For example, the support part 83 is bent in the vicinity of the distal end part of the support part 83. In this case, it is preferred that the pipe part 91 (the tube part 92) be formed of a flexible material such as silicone or rubber. Moreover, the nozzle unit 80 may include the curved rod part 81. In this case, the pipe part 91 is also curved similarly to the rod part 81. In this case, it is preferred that the pipe part 91 be configured to slide and move.

In the above description, the suction/drain unit includes the syringe 70 and the handpiece 10. However, the suction/drain unit is not limited to this, but it is sufficient if the suction/drain unit is configured to be capable of discharging a graft through the nozzle unit 80. The suction/drain unit may include, for example, a suction pump or a drain pump.

In the above description, the drain hole 83a as a fluid drain hole is provided to the front surface of the support part 83. However, the drain hole is not limited to this, but it is sufficient if the drain hole is configured to be capable of discharging the graft I to the outside of the nozzle unit 80. For example, the drain hole may be provided backward of the support part 83. In this case, a gap or groove is provided between the support part 83 and the tube part 92. The fluid is drained through the gap or groove.

In the above description, the rod part 81 includes the distal end part 82 and the support part 83. However, the rod part 81 is not limited to this, but may not include the support part 83. It is sufficient if the rod part 81 is configured to be capable of adhering the graft I thereto. For example, the rod part 81 may include a member having a similar outer diameter to the distal end part 82, instead of the support part 83. In this case, this member is supported by the proximal part 84.

In the above description, the support part 83 is provided to the rod part 81. However, a support part of the nozzle unit 80 is not limited to this, but may be provided to another portion. For example, the support part may be an inner side (interior wall) of the tube part 92. For example, the rod part 81 may be a rod-shaped member having a constant diameter. In this case, the rod part 81 may be configured to adhere the graft I to a distal end part thereof. In this case, a step height is provided to the inner diameter of the pipe part 91. The inner diameter of the pipe part 91 at a portion covering the distal end part of the rod part 81 is larger than the outer diameter of the distal end part of the rod part 81. Consequently, it is possible to ensure space to adhere the graft I in the distal end part of the rod part 81. Moreover, the inner diameter of the pipe part 91 at a portion other than the distal end part of the rod part 81 may be a diameter to the extent of equal to or slightly larger than the diameter of the rod part 81. Consequently, it becomes possible to ensure space to adhere the graft I in the distal end part of the rod part 81 and to make it hard for the pipe part 91 to rattle. In this manner, it is also possible to provide the support part to the pipe part 91 (the tube part 92). At this point, the support part of the pipe part 91 comes into contact with the rod part 81 and supports the pipe part 91.

In the above description, the suction/drain unit discharges the graft I. However, the surgical instrument 100 is not limited to this, but it is sufficient if the surgical instrument 100 is configured to be capable of adhering the graft I to the distal end part 82 and separating the graft I in an eye. The surgical instrument 100 may not include a member for sucking and draining fluid. In such a case, when the graft I is separated from the distal end part 82, it is preferred that the pipe part 91 be moved backward. Moreover, in such a case, a liquid flow passage, a drain hole, and the like become unnecessary.

In the above description, the nozzle unit 80 is one unit. Furthermore, the moving unit (the rotating part 95) is provided to the nozzle unit 80. However, they are not limited to this, but a mechanism being a moving unit may be provided to the handpiece 10. In this case, the rod part and the pipe part are attached to the handpiece 10 as individual members. Furthermore, the rod part and the pipe part act as attachments for a surgical instrument (components of the surgical instrument).

In the above description, the size of the graft I is 3 × 3 mm. However, the size of the graft I is not limited to this. When the size of the graft I is changed, it is sufficient if the configuration of the nozzle unit 80 is changed depending on the changed size. When the graft I is adhered to the distal end part 82, the graft I may overlap on itself. For example, the outer diameter of the distal end part 82 may be reduced, and the graft I may be overlapped double on itself (the graft I may be wound twice around the distal end part 82) to be adhered to the distal end part 82. Otherwise, the graft I may be overlapped on itself double or more than double.

In the above description, the graft I is a cell sheet of retinal pigment cells. However, the graft I is not limited to this, but it is sufficient if the graft I is fundus tissue. For example, the graft I may be a sheet of retinal cells.

In the above description, the graft I is one that has differentiated tissue removed from a patient. However, the graft I is not limited to this, but it is sufficient if the graft I is biological tissue. For example, the graft I may be a cell, tissue, or the like obtained from another living body. Moreover, the graft I may be, for example, a sheet that emits a substance having a treatment effect, a device that stimulates eyeball tissue, or a device that measures the state of eyeball tissue.

In the above description, the surgical instrument 100 is used in ophthalmic surgery. However, the surgical instrument 100 is not limited to this, but is applicable to arbitrary surgery to transplant (implant) the graft I in the body of a living body. For example, the surgical instrument 100 may be used in transplant surgery to a tissue other than an eyeball.

As described above, the present disclosure is not limited to the embodiment. Various modifications can be made to the present disclosure. The present disclosure includes also such modifications within a range where the technical idea is identical.

Moreover, the surgical instrument according to the embodiment may be the following first to seventh surgical instruments.

The first surgical instrument is a surgical instrument for implanting a graft in a patient, and includes a handpiece for an operator to hold, a rod-shaped rod part provided to a distal end of the handpiece and configured to adhere the graft to its side surface, a hollow pipe part configured to cover the rod part, the hollow pipe part having an inner diameter with predetermined clearance in between with the rod part, and a moving unit configured to move the pipe part relatively along an axial direction of the rod part.

The second surgical instrument in the first surgical instrument has a drain unit configured to drain a predetermined fluid from the rod part, and the rod part has a drain hole for the drain unit to drain the fluid to the outside. The third surgical instrument in the first surgical instrument has the rod part having a distal end part configured to adhere the graft thereto and a support part configured to support the distal end part, and the distal end part has a smaller diameter than the support part.

The fourth surgical instrument in the first surgical instrument has the rod part having a distal end with a longer circumference than the length of one side of the graft. The fifth surgical instrument in the fourth surgical instrument has the drain hole arranged on a side surface of the distal end part and/or on a distal end part side of the support part. The sixth surgical instrument in the first surgical instrument has the rod part having a circular shape in cross section orthogonal to the axial direction. The seventh surgical instrument in the first surgical instrument has the pipe part having a circular shape in cross section orthogonal to the axial direction. In the eighth surgical instrument in the first surgical instrument, the graft is biological tissue. In the ninth surgical instrument in the first surgical instrument, the graft is a small piece to be transplanted in the fundus of a patient's eye.

The foregoing detailed description has been presented for the purposes of illustration and description. Many modifications and variations are possible in light of the above teaching. It is not intended to be exhaustive or to limit the subject matter described herein to the precise form disclosed. Although the subject matter has been described in language specific to structural features and/or methodological acts, it is to be understood that the subject matter defined in the appended claims is not necessarily limited to the specific features or acts described above. Rather, the specific features and acts described above are disclosed as example forms of implementing the claims appended hereto.

## Claims

1. An ophthalmic surgical instrument (100) comprising:
a handpiece (10) and a syringe (70) inserted and fixed into the handpiece (10);
a rod part (81) at a distal end of the handpiece (10), the rod part (81) including a distal end part (82) having a surface for adhering a graft thereto; and
a hollow pipe part (91, 92) configured to cover the distal end part (82) of the rod part (81), the pipe part (91, 92) including an inner diameter with a predetermined clearance between the pipe part (91, 92) and the rod part (81); and
a moving unit (94, 95) configured to move the pipe part (91, 92) relative to the rod part (81) along an axial direction of the rod part (81) such that the distal end part (82) is exposed from the pipe part or the distal end part is covered with the pipe part (91, 92),
**characterized by**
a nozzle unit (80) including the rod part (81), the hollow pipe part (91, 92) and the moving unit (94, 95), wherein the nozzle unit (80) is fixed to a distal end part of the syringe (70).

2. The surgical instrument (100) according to claim 1, further comprising a drain unit (75) configured to drain a predetermined fluid from the rod part (81), wherein the rod part (81) includes a drain hole (82a) configured to drain the fluid to the outside.

3. The surgical instrument (100) according to claim 2, wherein the rod part (81) has a circular shape in cross section orthogonal to the axial direction,the rod part (81) includes the distal end part configured to adhere a graft thereto, and a support part (83) configured to support the distal end part (82), and
the distal end part (82) has a smaller outer diameter than the support part (83).

4. The surgical instrument (100) according to claim 3, wherein the drain hole (82a) is arranged on a surface of a distal end part (82).

5. The surgical instrument (100) according to claim 4, wherein the drain hole (82a) is arranged at a position to be covered by the graft upon adhering the graft to the distal end part (82).

6. The surgical instrument (100) according to any one of claims 3 to 5, wherein the drain hole (82a) is arranged on an end surface on a distal end part side of the support part (83).

7. The surgical instrument (100) according to any one of claims 3 to 6, wherein the distal end part (82) of the rod part (81) has a longer circumference than a length of one side of the graft.

8. The surgical instrument (100) according to any one of claims 3 to 7, wherein a step height greater than the thickness of the graft is provided between the distal end part (82) and the support part (83).

9. The surgical instrument (100) according to any one of claims 1 and 2, wherein the rod part (81) has a circular shape in cross section orthogonal to the axial direction.

10. The surgical instrument (100) according to any one of claims 1 to 9, wherein the pipe part (91, 92) has a circular shape in cross section orthogonal to the axial direction.

11. The surgical instrument (100) according to any one of claims 1 to 10, wherein the surface of the rod part (81) adheres to a biological tissue as the graft.

12. The surgical instrument (100) according to any one of claims 1 to 11, wherein the surface of the rod part (81) adheres to a small piece to be transplanted in the fundus of a patient's eye as the graft.

## Patentansprüche

1. Ein augenchirurgisches Instrument (100), das folgende Merkmale aufweist:
ein Handstück (10) und eine Spritze (70), die in das Handstück (10) eingeführt und darin befestigt ist;
einen Stabteil (81) an einem Distalende des Handstücks (10), wobei der Stabteil (81) einen Distalendteil (82) mit einer Oberfläche zum Haftbefestigen eines Transplantats an denselben umfasst; und
einen Hohlröhrenteil (91, 92), der ausgebildet ist, den Distalendteil (82) des Stabteils (81) zu bedecken, wobei der Röhrenteil (91, 92) einen Innendurchmesser mit einem vorbestimmten Zwischenraum zwischen dem Röhrenteil (91, 92) und dem Stabteil (81) umfasst; und
eine bewegliche Einheit (94, 95), die ausgebildet ist, den Röhrenteil (91, 92) relativ zu dem Stabteil (81) entlang einer Axialrichtung des Stabteils (81) derart zu bewegen, dass der Distalendteil (82) von dem Röhrenteil freigelegt ist oder der Distalendteil mit dem Röhrenteil (91, 92) bedeckt ist,
**gekennzeichnet durch**
eine Düseneinheit (80), die den Stabteil (81), den Hohlröhrenteil (91, 92) und die bewegliche Einheit (94, 95) umfasst, wobei die Düseneinheit (80) an einem Distalendteil der Spritze (70) befestigt ist.

2. Das chirurgische Instrument (100) gemäß Anspruch 1, das ferner eine Ableiteinheit (75) aufweist, die ausgebildet ist, ein vorbestimmtes Fluid aus dem Stabteil (81) abzuleiten, wobei der Stabteil (81) ein Ableitloch (82a) umfasst, das ausgebildet ist, das Fluid nach außen abzuleiten.

3. Das chirurgische Instrument (100) gemäß Anspruch 2, bei dem der Stabteil (81) in einem Querschnitt, der orthogonal zu der Axialrichtung ist, eine kreisförmige Gestalt aufweist, wobei der Stabteil (81) den Distalendteil, der ausgebildet ist, ein Transplantat an denselben haften zu lassen, und einen Trägerteil (83), der ausgebildet ist, den Distalendteil (82) zu tragen, umfasst, und
der Distalendteil (82) einen kleineren Außendurchmesser hat als der Trägerteil (83).

4. Das chirurgische Instrument (100) gemäß Anspruch 3, bei dem das Ableitloch (82a) auf einer Oberfläche eines Distalendteils (82) angeordnet ist.

5. Das chirurgische Instrument (100) gemäß Anspruch 4, bei dem das Ableitloch (82a) an einer Position angeordnet ist, die beim Anhaften des Transplantats an den Distalendteil (82) durch das Transplantat zu bedecken ist.

6. Das chirurgische Instrument (100) gemäß einem der Ansprüche 3 bis 5, bei dem das Ableitloch (82a) auf einer Endoberfläche an einer Distalendteilseite des Trägerteils (83) angeordnet ist.

7. Das chirurgische Instrument (100) gemäß einem der Ansprüche 3 bis 6, bei dem der Distalendteil (82) des Stabteils (81) einen Umfang hat, der länger als eine Länge einer Seite des Transplantats ist.

8. Das chirurgische Instrument (100) gemäß einem der Ansprüche 3 bis 7, bei dem eine Stufenhöhe, die größer als die Dicke des Transplantats ist, zwischen dem Distalendteil (82) und dem Trägerteil (83) vorgesehen ist.

9. Das chirurgische Instrument (100) gemäß einem der Ansprüche 1 und 2, bei dem der Stabteil (81) in einem Querschnitt, der orthogonal zu der Axialrichtung ist, eine kreisförmige Gestalt aufweist.

10. Das chirurgische Instrument (100) gemäß einem der Ansprüche 1 bis 9, bei dem der Röhrenteil (91, 92) in einem Querschnitt, der orthogonal zu der Axialrichtung ist, eine kreisförmige Gestalt aufweist.

11. Das chirurgische Instrument (100) gemäß einem der Ansprüche 1 bis 10, bei dem die Oberfläche des Stabteils (81) an einem biologischen Gewebe als Transplantat haftet.

12. Das chirurgische Instrument (100) gemäß einem der Ansprüche 1 bis 11, bei dem die Oberfläche des Stabteils (81) an einem kleinen Stück haftet, das als Transplantat in den Augenhintergrund eines Patienten transplantiert werden soll.

## Revendications

1. Instrument chirurgical ophtalmique (100), comprenant:
une pièce à main (10) et une seringue (70) introduite et fixée dans la pièce à main (10);
une partie de tige (81) à une extrémité distale de la pièce à main (10), la partie de tige (81) comportant une partie d'extrémité distale (82) présentant une surface destinée à y adhérer un greffon; et
une partie de tube creux (91, 92) configurée pour couvrir la partie d'extrémité distale (82) de la partie de tige (81), la partie de tube (91, 92) comportant un diamètre intérieur avec un jeu prédéterminé entre la partie de tube (91, 92) et la partie de tige (81); et
une unité de déplacement (94, 95) configurée pour déplacer la partie de tube (91, 92) par rapport à la partie de tige (81) dans une direction axiale de la partie de tige (81) de sorte que la partie d'extrémité distale (82) soit exposée par rapport à la partie de tube ou que la partie d'extrémité distale soit couverte par la partie de tube (91, 92),
**caractérisé par**
une unité de buse (80) comportant la partie de tige (81), la partie de tube creux (91, 92) et l'unité de déplacement (94, 95), dans lequel l'unité de buse (80) est fixée à une partie d'extrémité distale de la seringue (70).

2. Instrument chirurgical (100) selon la revendication 1, comprenant par ailleurs une unité de vidange (75) configurée pour évacuer un fluide prédéterminé de la partie de tige (81), dans lequel la partie de tige (81) comporte un trou d'évacuation (82a) configuré pour évacuer le liquide vers l'extérieur.

3. Instrument chirurgical (100) selon la revendication 2, dans lequel la partie de tige (81) présente une section de forme circulaire orthogonale à la direction axiale, la partie de tige (81) comporte la partie d'extrémité distale configurée pour y adhérer un greffon, et une partie de support (83) configurée pour supporter la partie d'extrémité distale (82), et
la partie d'extrémité distale (82) présente un diamètre extérieur plus petit que la partie de support (83).

4. Instrument chirurgical (100) selon la revendication 3, dans lequel le trou d'évacuation (82a) est disposé sur une surface d'une partie d'extrémité distale (82).

5. Instrument chirurgical (100) selon la revendication 4, dans lequel le trou d'évacuation (82a) est disposé en une position à couvrir par le greffon après adhérence du greffon à la partie d'extrémité distale (82).

6. Instrument chirurgical (100) selon l'une quelconque des revendications 3 à 5, dans lequel le trou d'évacuation (82a) est disposé sur une surface d'extrémité d'un côté de la partie d'extrémité distale de la partie de support (83).

7. Instrument chirurgical (100) selon l'une quelconque des revendications 3 à 6, dans lequel la partie d'extrémité distale (82) de la partie de tige (81) présente une circonférence plus longueur qu'une longueur d'un côté du greffon.

8. Instrument chirurgical (100) selon l'une quelconque des revendications 3 à 7, dans lequel une hauteur d'étage supérieure à l'épaisseur du greffon est prévue entre la partie d'extrémité distale (82) et la partie de support (83).

9. Instrument chirurgical (100) selon l'une quelconque des revendications 1 et 2, dans lequel la partie de tige (81) présente une section de forme circulaire perpendiculaire à la direction axiale.

10. Instrument chirurgical (100) selon l'une quelconque des revendications 1 à 9, dans lequel la partie de tube (91, 92) présente une section de forme circulaire perpendiculaire à la direction axiale.

11. Instrument chirurgical (100) selon l'une quelconque des revendications 1 à 10, dans lequel la surface de la partie de tige (81) adhère à un tissu biologique comme greffon.

12. Instrument chirurgical (100) selon l'une quelconque des revendications 1 à 11, dans lequel la surface de la partie de tige (81) adhère à une petite pièce à transplanter dans le fond de l'oeil d'un patient comme greffon.
